# EUROPEAN PATENT APPLICATION

(11) **EP 3 109 314 A1**
(43) Date of publication of application: **28.12.2016**
(21) Application number: 15748738.0
(22) Date of filing: 17.02.2015
(51) Int. Cl.: C12M 3/02, C12M 1/02

(54) **CELL CULTURING DEVICE**

(30) Priority: 17.02.2014 JP 2014027565
(71) Applicant: Asahi Kasei Kabushiki Kaisha, Chiyoda-ku Tokyo 101-8101 (JP); Tokyo Women's Medical University, Tokyo 162-8666 (JP)
(72) Inventor: KONISHI Kanako, Tokyo 101-8101 (JP); IWAMOTO Ushio, Tokyo 101-8101 (JP); WADA Masanori, Tokyo 162-0813 (JP); MATSUURA Katsuhisa, Tokyo 162-8666 (JP); SHIMIZU Tatsuya, Tokyo 162-8666 (JP); OKANO Teruo, Tokyo 162-8666 (JP)
(74) Representative: dompatent von Kreisler Selting Werner - Partnerschaft von Patent- und Rechtsanwälten mbB
(86) International application number: PCT/JP2015/054222
(87) International publication number: WO 2015/122528

(57) **Abstract**

A cell culturing device is provided that allows satisfactory culturing of cells with a simple configuration. A cell culturing device 5 includes a culture tank 15 to accommodate culture solution containing a cell, a shaft member 17 at least partially disposed in the culture tank 15, a stirring mechanism 19 supported by the shaft member 17, disposed in the culture tank 15, and having at least a pair of stirring blades 27 configured to be rotatable about the shaft member 17, and a filter 21 disposed in contact with the shaft member 17 so as to suck the culture solution from the culture tank 15 and/or to supply the culture solution to the culture tank 15. The shaft member 17 is at least partially hollow, has openings 17a, 17b to introduce the culture solution to the interior or to discharge the culture solution from the interior, and is unrotatable. Suction of the culture solution from the culture tank 15 and/or supply of the culture solution to the tank 15 is performed through the filter 21 and the interior of the shaft member 17.

## Description

### Technical Field

The present invention relates to a cell culturing device.

### Background Art

In the cell culturing field and the regenerative medicine field, techniques (cell culturing devices) have recently been in demand for culturing a large amount of cells automatically and stably for a long time to obtain products of cells usable for biological products and cells themselves for use in regenerative tissue construction. In order to stably culture cells for a long time, it is often necessary to change and/or clean biological solutions during culture, and add necessary components and remove unnecessary components. In doing so, a technique for separating cells from culture solution and removing culture solution alone is required in order not to affect the state of cells. An example of conventional cell culturing devices for this purpose is known to be described in Patent Literature 1. The cell culturing device described in Patent Literature 1 has a disk-shaped body at least partially having a filter region that allows culture solution to pass through but does not allow cells to pass through. This disk-shaped body has a conduit for drawing culture solution to the outside and is provided to be rotatable concentrically with the conduit.

### Citation List

### Patent Literature

[Patent Literature 1] Japanese Unexamined Patent Publication No. S60-224486

### Summary of Invention

### Technical Problem

Examples of cells cultured in cell culturing devices include human iPS (induced pluripotent stem) cells. Human iPS cells are prone to die when the cells are brought into a single cell state or exposed to high shear stress. Thus, when human iPS cells are cultured, shear stress on cells should be minimized in the cell culturing device. In the device described in the patent literature above, a filter that sucks culture solution is mounted on the disk-shaped body serving as stirring means. The conventional device therefore has no obstacle disturbing a solution flow and is effective in reducing stress on cells, when compared with a configuration separately including a system for sucking culture solution. However, the conventional device is configured such that the sucking mechanism and the stirring mechanism rotate at the same time, and thus a configuration for securing sealability is required in the rotation mechanism, which complicates the configuration.

An object of the present invention is to provide a cell culturing device that allows satisfactory culturing of cells with a simple configuration.

### Solution to Problem

A cell culturing device according to an aspect of the present invention includes a culture tank configured to accommodate culture solution containing a cell, a shaft member at least partially disposed in the culture tank, stirring means supported by the shaft member and disposed in the culture tank, the stirring means having a stirring blade configured to be rotatable about the shaft member, and a filter disposed in contact with the shaft member. The filter allows the culture solution to pass through and does not allow the cell to pass through. The shaft member is at least partially hollow, has an opening configured to introduce the culture solution to the interior of the shaft member or to discharge the culture solution from the interior, and is configured to be unrotatable. The filter is located inner than a movable region of the stirring blade rotating about the shaft member and is disposed on the shaft member independently of the stirring blade. Suction of the culture solution from the culture tank and/or supply of the culture solution to the culture tank is performed through the filter and the interior of the shaft member.

In this cell culturing device, the filter is provided on the shaft member configured to be unrotatable. The stirring blade of the stirring means is provided to be rotatable about the shaft member. In this manner, in the cell culturing device, the stirring blade rotates relative to the shaft member, whereas the shaft member and the filter do not rotate. Thus, in the cell culturing device, because the mechanism for sucking and/or supplying culture solution is not rotated, a simple configuration can be achieved. In the cell culturing device, the filter sucking culture solution from the culture tank and/or supplying culture solution to the culture tank is located inner than the movable region of the stirring blade on the shaft member about which the stirring blade rotates. Suction of culture solution from the culture tank and/or supply of culture solution to the culture tank is performed through the filter and the interior of the shaft member. In the cell culturing device, disturbance by the filter in the solution flow (laminar flow) produced by the rotation of the stirring blade is thus suppressed, thereby reducing stress on cells in connection with suction and/or supply of culture solution. In the cell culturing device, therefore, culturing of cells can be performed satisfactorily with a simple configuration.

In an embodiment, the shaft member may be supported by a bottom of the culture tank.

In an embodiment, the shaft member may have a lower end having the opening and have an upper end closed. The shaft member may have a side surface having an opening in communication with the interior. The filter may be disposed on an outer surface of the shaft member so as to cover the opening at the side surface. In this way, in the cell culturing device, the mechanism for sucking and/or supplying culture solution is integrated to the shaft member, and the shaft member does not rotate. Simplification of the device thus can be achieved.

In an embodiment, the filter may have a tubular shape and extend on outside of the shaft member so as to cover the opening at the side surface. With this configuration, in the cell culturing device, the surface area (membrane area) of the filter can be secured.

In an embodiment, the shaft member may have a tubular shape that has a lower end having the opening and that has an upper end having an opening in communication with the interior. The filter may be disposed at the shaft member so as to cover the opening at the upper end. In this way, in the cell culturing device, the mechanism for sucking and/or supplying culture solution is integrated to the shaft member, and the shaft member does not rotate. Simplification of the device thus can be achieved.

In an embodiment, the filter may have a tubular shape with a base and extend on outside of the shaft member so as to cover the opening at the upper end. With this configuration, in the cell culturing device, the surface area (membrane area) of the filter can be secured.

In an embodiment, the shaft member may be supported by a lid of the culture tank.

In an embodiment, the shaft member may have a tubular shape having an upper end having the opening and a lower end having an opening in communication with the interior. The filter may be disposed on the shaft member so as to cover the opening at the lower end. In this way, in the cell culturing device, the mechanism for sucking and/or supplying culture solution is integrated to the shaft member, and the shaft member does not rotate. Simplification of the device thus can be achieved.

In an embodiment, the filter may have a tubular shape with a base and extend on outside of the shaft member so as to cover the opening at the lower end. With this configuration, in the cell culturing device, the surface area (membrane area) of the filter can be secured.

In an embodiment, the shaft member may have an upper end having the opening and a lower end closed. The shaft member may have a side surface having an opening in communication with the interior. The filter may be disposed on an outer surface of the shaft member so as to cover the opening at the side surface. In this way, in the cell culturing device, the mechanism for sucking and/or supplying culture solution is integrated to the shaft member, and the shaft member does not rotate. Simplification of the device thus can be achieved.

In an embodiment, the filter may have a tubular shape and extend on outside of the shaft member so as to cover the opening at the side surface. With this configuration, in the cell culturing device, the surface area (membrane area) of the filter can be secured.

In an embodiment, the filter may be porous.

### Advantageous Effects of Invention

The present invention allows satisfactory culturing of cells with a simple configuration.

### Brief Description of Drawings

[FIG. 1] FIG. 1 is a diagram showing a cell culturing system including a cell culturing device according to a first embodiment.
[FIG. 2] FIG. 2 is a perspective view showing the cell culturing device.
[FIG. 3] FIG. 3 is a side view of a culture tank shown in FIG. 2.
[FIG. 4] FIG 4 is a diagram showing a cross-sectional configuration along the line IV-IV in FIG. 3.
[FIG. 5] FIG. 5 is a side view showing a usage state of the cell culturing device.
[FIG. 6] FIG. 6 is a diagram showing a modification of the cell culturing device according to the first embodiment.
[FIG. 7] FIG. 7 is a diagram showing another embodiment of the cell culturing system including the cell culturing device.
[FIG. 8] FIG. 8 is a diagram showing another embodiment of the cell culturing system including the cell culturing device.
[FIG. 9] FIG. 9 is a diagram showing a cross-sectional configuration of a cell culturing device according to a second embodiment.
[FIG. 10] FIG. 10 is a diagram showing a modification of the cell culturing device according to the second embodiment.
[FIG. 11] FIG. 11 is a diagram showing a modification of the cell culturing device according to the second embodiment.
[FIG. 12] FIG. 12 is a diagram showing a modification of the cell culturing device according to the second embodiment.
[FIG 13] FIG. 13 is a diagram showing a modification of the cell culturing device according to the second embodiment.
[FIG. 14] FIG. 14 is a diagram showing a modification of the cell culturing device according to the second embodiment.
[FIG. 15] FIG. 15 is a diagram showing a cell culturing device according to Example 1.
[FIG. 16] FIG. 16 is a diagram showing a cell culturing device according to Example 2.
[FIG. 17] FIG 17 is a diagram showing a cell culturing device according to Comparative Example 1.

### Description of Embodiments

Preferred embodiments of the present invention will be described in detail below with reference to the accompanying drawings. It should be noted that in the description of the drawings, the same or corresponding elements are denoted with the same reference signs and an overlapping description will be omitted.

### First Embodiment

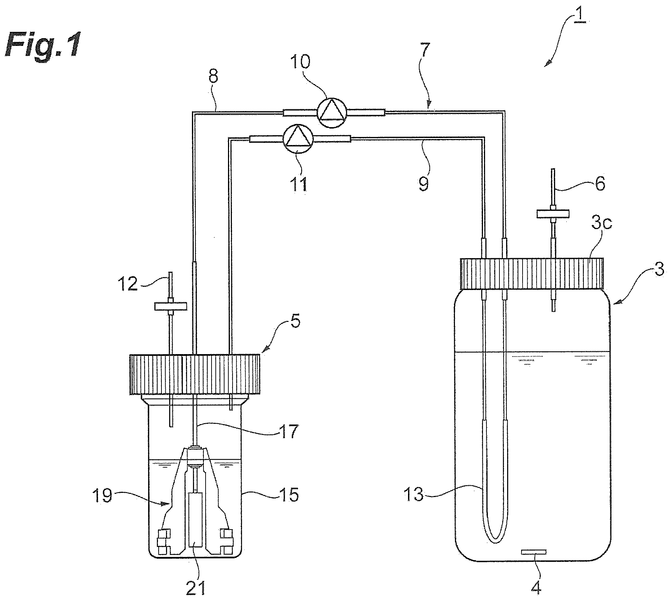

FIG. 1 is diagram showing a cell culturing system including a cell culturing device according to a first embodiment. In the cell culturing system 1 shown in FIG. 1, cells are cultured. The cells are useful cells such as mammalian cells. When a product of cells is used as a result of culture, cells likely to produce a substance to be used or cells in which a particular gene is introduced to facilitate production of the substance of interest can be selected as the cells. Alternatively, when particular cells are used as a result of culture, cells or others genetically modified to facilitate proliferation of the cells may be used as the cells.

Alternatively, mature cells or undifferentiated cells may be used as the cells without being limited by maturity. Examples of the cells include cells taken from body tissues and subjected to enzymatic treatment, cells derived from blood, mesenchymal stem cells, ES (Embryonic stem) cells, iPS (induced pluripotent stem) cells, and cells differentiation-induced from these cells. The cells are not limited to adhesion cells or suspension cells, or by any particular culture method. Examples of suspension culture include suspension culture of single cells, suspension culture of cell condensations, and suspension culture of cells carried on minute carriers. The cells are not limited to a single kind of cells. The system may be applicable to co-culture in which other cells are mixed for producing a substance that facilitates growth of cells of interest.

A configuration of the cell culturing system 1 will now be described. As shown in FIG. 1, the cell culturing system 1 includes a component-controlling solution tank 3, a cell culturing device 5, and a feed circuit 7. The component-controlling solution tank 3 and the cell culturing device 5 are connected through the feed circuit 7. The feed circuit 7 feeds culture solution, which will be described later, between the component-controlling solution tank 3 and the cell culturing device 5. The feed circuit 7 includes two tubes 8 and 9 and feed pumps 10 and 11. The tubes 8 and 9 have a hollow structure to be able to sterilely feed culture solution. The tubes 8 and 9 are formed of a material such as silicone, urethane, fluoropolymer, or polyvinyl chloride.

The feed pumps 10 and 11 are provided on the feed paths of the tubes 8 and 9, respectively, and are able to continuously feed culture solution. The feed pumps 10 and 11 may be any common pumps, for example, Perista pumps or diaphragm pumps. The feed circuit 7 is driven by the feed pumps 10 and 11 to feed culture solution between the component-controlling solution tank 3 and the cell culturing device 5 through the tubes 8 and 9.

The tubes 8 and 9 of the feed circuit 7 are provided with a culture solution component-controlling membrane 13. Specifically, the culture solution component-controlling membrane 13 is connected to one end of the tube 8 and to one end of the tube 9 and is disposed in the component-controlling solution tank 3. The culture solution component-controlling membrane 13 is a semi-permeable membrane having permeability to a culture solution component, the permeability being dependent on the molecular weight of the component. The pore size of the culture solution component-controlling membrane 13 is designed depending on the molecular weight of the component intended to be held in the cell culturing device 5. That is, the culture solution component-controlling membrane 13 is selected such that, among the components intended to be held in the cell culturing device 5, the smallest molecular weight substance does not pass through the membrane. The culture solution component-controlling membrane 13 may be formed in a flat sheet shape or a hollow fiber shape. For the purpose of conveying culture solution, a hollow fiber shape is preferred. In order to efficiently perform component control, it is further preferable to use a plurality of hollow fibers formed into a bundle. In FIG. 1, the culture solution component-controlling membrane 13 formed in a hollow fiber shape is illustrated.

The material of the culture solution component-controlling membrane 13 is preferably, but not limited to, a material that does not allow adsorption or decomposition of the components intended to be held in the cell culturing device 5. When the material of the culture solution component-controlling membrane 13 has a property of easily adsorbing the above-noted components, the culture solution component-controlling membrane 13 may be coated with other materials or a surface modifier may be used to modify the surface to suppress adsorption of the components.

The component-controlling solution tank 3 is a tank that accommodates a component-controlling solution. The component-controlling solution tank 3 is preferably formed of a material that is inert to the component-controlling solution components, does not have cytotoxicity, and is resistant to sterilization (including decontamination, disinfection, and asepsis) treatment. Examples of the material include glasses, synthetic resins, and stainless steels. The internal capacity, the shape, and the like of the component-controlling solution tank 3 are appropriately determined according to the amount of the component-controlling solution. In the present embodiment, the component-controlling solution tank 3 is made of, for example, glass and is shaped like a cylinder with a base. The component-controlling solution tank 3 is provided with a lid 3c for closing an opening (not shown) provided on the top.

The component-controlling solution accommodated in the component-controlling solution tank 3 is a solution containing at least one of components that can substantially pass through the culture solution component-controlling membrane 13. The components contained in the culture solution and the component-controlling solution are controlled through the membrane depending on their molecular weights and the difference in concentration between those solutions.

A component that has a molecular weight greater than the pore size of the culture solution component-controlling membrane 13 and cannot substantially pass through the membrane does not move between the solutions. By contrast, a component that has a molecular weight smaller than the pore size of the culture solution component-controlling membrane 13 and can substantially pass through the membrane has its concentration controlled between the solutions such that the difference in concentration is reduced. Metabolites produced from cells and accumulated in the culture solution move toward the component-controlling solution so that the concentration of the metabolites in the culture solution is reduced. At the same time, the component necessary for cell growth and reduced in concentration during culture moves from the component-controlling solution and is added to the culture solution. Based on the principle described above, the environment in the culture solution is maintained and a good growth environment for cells is maintained by appropriately setting the content and concentration of the component-controlling solution. The culture solution can be used as it is, as a matter of course. Thus, the component-controlling solution preferably contains all the components to be lost from the culture solution during cell culture. More preferably, the concentrations of the components are set such that the components are not depleted during cell culture.

The amount of the component-controlling solution is preferably set large in view of preventing accumulation of cell metabolites. Preferably, the amount of the component-controlling solution is set at least 5 times, more preferably at least 10 times, the amount of the culture solution. The amount of the component-controlling solution, which affects the culture costs, is determined by the culture period and the required number of cells. The component-controlling solution may be designed to have buffer capacity to facilitate maintenance of physiological pH or may be mixed with a pH indicator pigment to facilitate identification of a pH change by color.

In the interior of the component-controlling solution tank 3, a stirring rotor 4 is provided. The stirring rotor 4 is a member for stirring the component-controlling solution in the component-controlling solution tank 3 and is driven by a not-shown drive source disposed below the component-controlling solution tank 3. The drive source is, for example, the one that drives rotation of a magnetic force element. The stirring rotor 4 rotates in response to the rotation drive of the magnetic force element to stir the component-controlling solution. The lid 3c of the component-controlling solution tank 3 may be provided with, for example, an air supply/exhaust port 6 and a sampling port (not shown) for sampling the component-controlling solution.

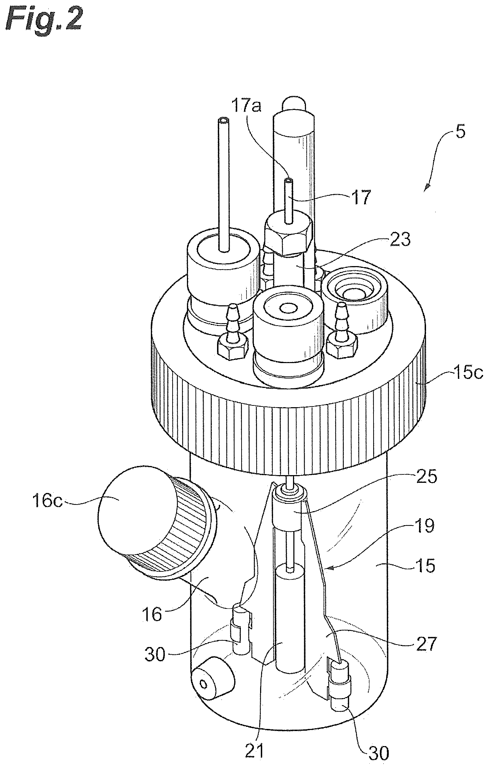

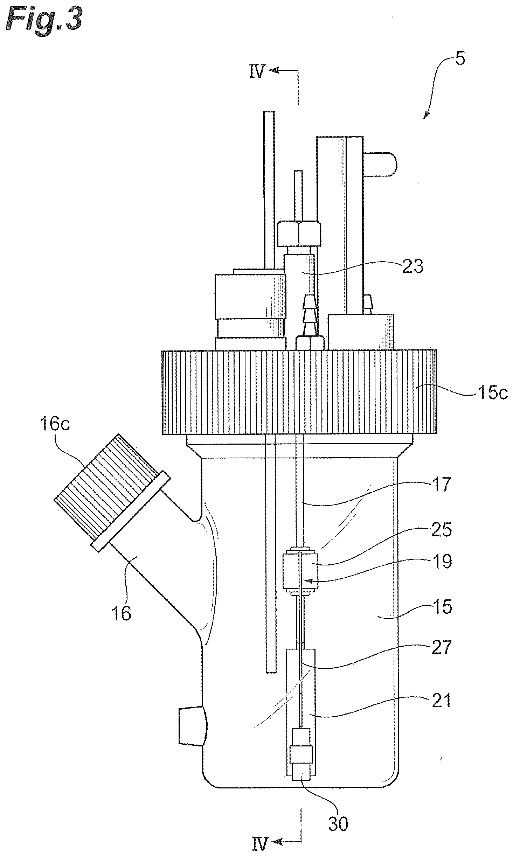

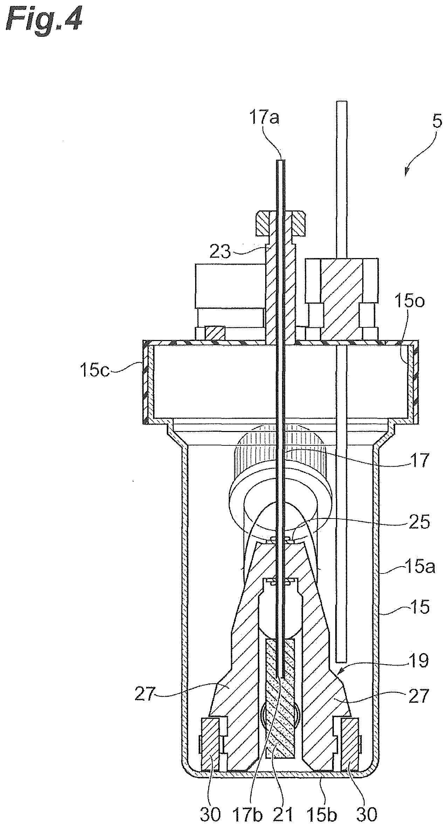

The cell culturing device 5 is a device for culturing cells. FIG. 2 is a perspective view showing the cell culturing device. FIG. 3 is a side view of the cell culturing device. FIG. 4 is a diagram showing a cross-sectional configuration along the line IV-IV in FIG 3. As shown in FIG. 2 to FIG 4, the cell culturing device 5 includes a culture tank 15, a shaft member 17, a stirring mechanism (stirring means) 19, and a filter 21.

The culture tank 15 accommodates (stores) culture solution in which cells are cultured. Here, the culture solution is a solution at least containing a variety of cells or others, and solution components and environment capable of growing the cells. The components and the concentration of the culture solution are designed depending on the properties of cells. The culture solution may be designed to have buffer capacity to facilitate maintenance of physiological pH or may be mixed with a pH indicator pigment to facilitate identification of a pH change by color. Any of commercially available culture solutions may be used as it is as the culture solution, or an additional component may be added thereto depending on the properties of cells of interest.

The culture tank 15 is preferably formed of a material that is inert to the culture solution components, does not have cytotoxicity, and is resistant to sterilization treatment. Examples of the material include glasses, synthetic resins, and stainless steels. The internal capacity, the shape, and the like of the culture tank 15 are appropriately determined according to the amount of the culture solution. In the present embodiment, the culture tank 15 is made of, for example, glass and is shaped like a cylinder with a base. The culture tank 15 has an outlet 16 for drawing the culture solution containing cells or others. The outlet 16 is a tubular body provided at a side surface 15a (see FIG. 4) of the culture tank 15 and extending obliquely upward. A lid 16c is attached to the outlet 16. The culture tank 15 is provided with a lid 15c for closing an opening 15o provided on the top. The lid 15c is provided with, for example, an air supply/exhaust port 12, a culture solution supply portion for supplying culture solution to the culture tank 15, and an insertion port for a pH sensor. The upper end of the culture solution supply portion is connected with the other end of the tube 9.

The shaft member 17 is a linear member and has a hollow structure. The shaft member 17 is preferably formed of a material that is inert to culture solution components, does not have cytotoxicity, and is resistant to sterilization treatment. Examples of the material include synthetic resins and stainless steels. The shaft member 17 has openings 17a and 17b at both ends, respectively.

The shaft member 17 has one end side disposed in the culture tank 15. Specifically, the shaft member 17 is fixed to a shaft support 23. The shaft support 23 is provided on the lid 15c. The shaft support 23 is disposed approximately at the center of the lid 15c and stands upright on the lid 15c. The shaft support 23 has a hollow structure and allows the shaft member 17 to pass through the interior of the shaft support 23 to unrotatably hold the shaft member 17. The other end of the shaft member 17 protrudes above the upper end surface of the shaft support 23. The upper end of the shaft member 17 is connected with the other end of the tube 8. The interior of the shaft member 17 is thus in communication with the tube 8.

The stirring mechanism 19 includes an attachment 25 and stirring blades 27. The attachment 25 is rotatably attached to the shaft member 17. The attachment 25 has the shaft member 17 inserted therethrough and is rotatably supported at a predetermined height position of the shaft member 17.

The stirring blades 27 stir the culture solution. The stirring blades 27 are provided to be rotatable about the shaft member 17. Specifically, the attachment 25 is coupled to the upper ends of the stirring blades 27, and the attachment 25 is rotatable relative to the shaft member 17, whereby the stirring blades 27 rotate about the shaft member 17. The stirring blades 27 are preferably formed of a plate material inert and resistant to the culture solution. Examples of the material include thin plate-shaped synthetic resins and stainless steels (for example, SUS 316 having a thickness of 1 mm). The number of stirring blades 27 is set depending on the number of revolutions of the stirring mechanism 19. The stirring blades 27 are preferably disposed at regular intervals around the shaft member 17 in view of balance during stirring. Preferably, for example, two to four stirring blades 27 are provided. In the present embodiment, a configuration including two stirring blades 27 is illustrated.

Each of the stirring blades 27 is formed such that the gap between the stirring blade 27 and the inner surface of a bottom 15b (see FIG. 4) of the culture tank 15 is small. The stirring blade 27 thus moves along the inner surface of the bottom 15b when the stirring mechanism 19 rotates. With this configuration, the culture solution in the vicinity of the inner surface of the bottom 15b is stirred so that cells, cell aggregations, and the like are continuously floated in the culture solution, thereby preventing precipitation on the inner surface of the bottom 15b. The stirring blade 27 is formed such that the gap between the stirring blade 27 and the inner surface of the side surface 15a of the culture tank 15 is small. The stirring blade 27 thus moves along the inner surface of the side surface 15a when the stirring mechanism 19 rotates. With this configuration, the culture solution in the vicinity of the inner surface of the side surface 15a is stirred to form a uniform laminar flow, thereby preventing precipitation of cell aggregations and the like, which increase in specific gravity with cell proliferation.

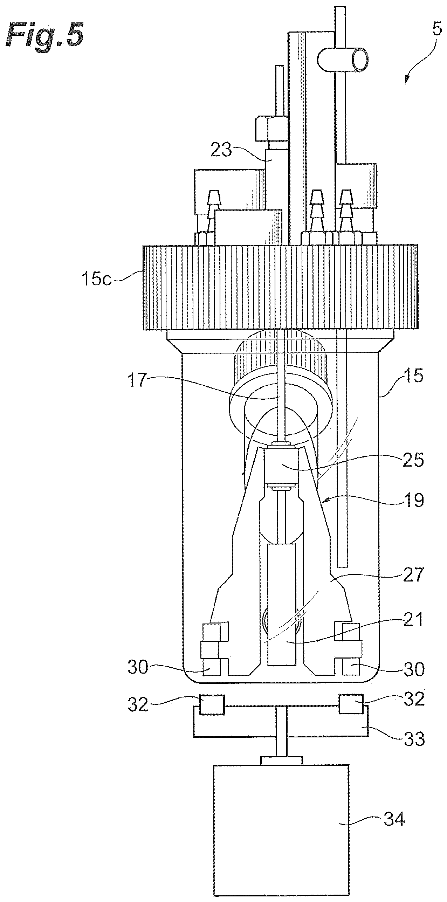

The stirring blade 27 is provided with a magnetic force element 30. The magnetic force element 30 is disposed at the lower end of the stirring blade 27. The magnetic force element 30 is, for example, a permanent magnet coated with, for example, tetrafluoroethylene. The magnetic force element 30 is fixed to a portion bent so as to surround the magnetic force element 30 at the lower end of the stirring blade 27. The stirring blade 27 is rotated by driving of the magnetic force element 30. Specifically, as shown in FIG. 5, a drive motor 34 is disposed below the culture tank 15 to drive the rotation of a pair of magnetic force elements 32 through a support 33. The stirring blades 27 rotate with the rotation of the magnetic force elements 32 disposed so as to face the magnetic force elements 30. The shaft of the drive motor 34 is disposed concentrically with the shaft member 17.

The filter 21 is a part in contact with the culture solution and allows the culture solution to pass through but does not allow the cells, the cell aggregations, and the like in the culture solution to pass through. The filter 21 may be any filter that can separate the cells and others from the culture solution, and the material, the shape, and the number thereof are not limited and can be appropriately set in accordance with the design. The filter 21 is, for example, porous, preferably, sintered and cylindrical. Examples of the material of the filter 21 include metals, ceramics, glasses, resins, and fibers. Preferable examples include fluoropolymers and polyolefin resins. The filter 21 is preferably formed of a material that allows water to pass through to some extent and that does not cause adsorption of cells themselves or minute carriers, or decomposition or adsorption of the components intended to be held in the cell culturing device 5. When the material of the filter 21 has such characteristics, the filter 21 may be coated with another material or a surface modifier may be used to achieve hydrophilicity or prevent the adsorption and decomposition. The filter 21 is provided in contact with the shaft member 17. Specifically, the filter 21 is shaped like a cylinder with a base and has the lower end of the shaft member 17 inserted therein. The filter 21 thus extends concentrically with the shaft member 17 and is located between the stirring blades 27 of the stirring mechanism 19. That is, the filter 21 is located inner than the movable region of the stirring blades 27 rotating about the shaft member 17 and is provided on the shaft member 17 independently of the stirring blades 27. The culture solution that has passed through the filter 21 is led to the interior of the shaft member 17.

In the cell culturing device 5, the culture solution sucked through the filter 21 is introduced from the opening 17b of the shaft member 17 to the interior thereof, discharged from the opening 17a, and led to the tube 8. The culture solution is then sent to the culture solution component-controlling membrane 13 in the component-controlling solution tank 3 through the tube 8 and is returned to the culture tank 15 through the tube 9.

As described above, the cell culturing device 5 in the present embodiment includes the filter 21 provided on the shaft member 17 configured to be unrotatable. The stirring blades 27 of the stirring mechanism 19 are provided to be rotatable about the shaft member 17. In this manner, in the cell culturing device 5, the stirring blades 27 rotate relative to the shaft member 17, whereas the shaft member 17 and the filter 21 do not rotate. Thus, in the cell culturing device 5, because the mechanism for sucking culture solution is not rotated, a simple configuration can be achieved. In the cell culturing device 5, the filter 21 sucking culture solution from the culture tank 15 is located inner than the movable region of the stirring blades 27 on the shaft member 17 about which the stirring blades 27 rotate, and the culture solution is sucked from the culture tank 15 through the filter 21 and the interior of the shaft member 17. In the cell culturing device 5, disturbance by the filter 21 in the solution flow (laminar flow) produced by the rotation of the stirring blades 27 thus can be suppressed, thereby reducing stress on cells in connection with suction of the culture solution. In the cell culturing device 5, therefore, culturing of cells can be performed satisfactorily with a simple configuration.

In the present embodiment, the shaft member 17 is supported by the shaft support 23 provided on the lid 15c, and the lower end of the shaft member 17 is inserted in the filter 21. In this way, in the cell culturing device 5, the mechanism for sucking culture solution is integrated to the shaft member 17, and the shaft member 17 does not rotate. Simplification of the device thus can be achieved.

In the present embodiment, the filter 21 has a column shape, which ensures the membrane area on the filter 21. Thus, the filter 21 can stably suck culture solution.

The cell culturing device 5 according to the first embodiment may simply be configured such that the shaft member is supported by the lid 15c of the culture tank 15. The configuration of the shaft member, the filter, and the stirring mechanism may be in different forms.

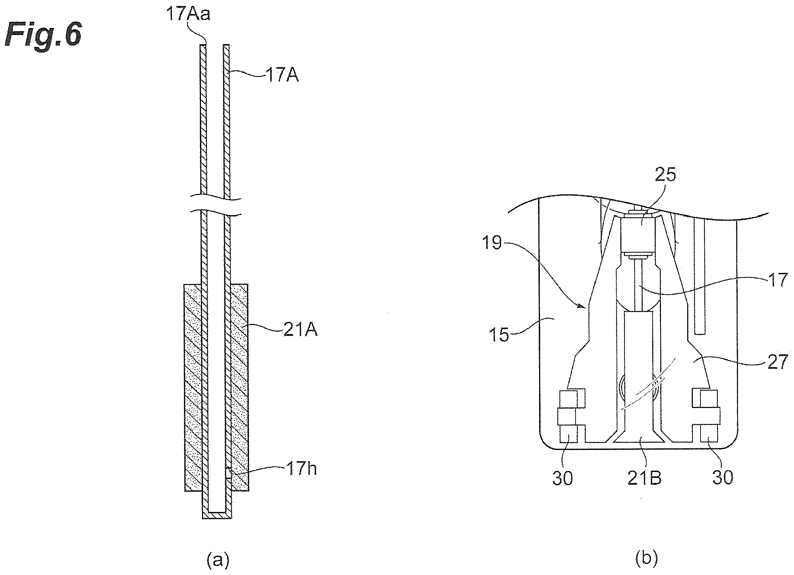

FIG. 6 is a diagram showing a modification of the cell culturing device according to the first embodiment. As shown in FIG. 6(a), a filter 21A may be configured such that a shaft member 17A is inserted therethrough. Specifically, the shaft member 17A has a hollow structure, has an opening 17Aa at the upper end, and is closed at the lower end. At a side surface of the shaft member 17A, an opening 17h is provided to communicate the interior with the exterior. Although FIG. 6 shows a single opening 17h, the number, the size, and the shape of the opening 17h can be appropriately set in accordance with the design. The filter 21A has a cylindrical shape. The filter 21A has the shaft member 17A inserted therethrough and is provided on the outer surface (outside) of the shaft member 17A so as to cover the opening 17h of the shaft member 17A. With this configuration, the culture solution is sucked from the culture tank 15 through the filter 21A and the interior of the shaft member 17A.

The filter 21 may have a shape other than a cylindrical shape. As shown in FIG 6(b), a filter 21B may be shaped like a truncated cone at the lower end. The filter 21 may be shaped like a prism or a triangular prism. The filter 21 may have a surface shape having projections and depressions in order to secure a surface area.

The filter 21 may be in contact with the bottom 15b of the culture tank 15. When the filter 21 is in contact with the bottom 15b, the vicinity of the center of the bottom is occupied by the filter 21. This configuration eliminates a space for stagnation of the solution flow in the vicinity of the center of the bottom due to rotation of the culture solution and prevents precipitation of cell aggregations and the like, which has increased in specific gravity with cell proliferation, in the vicinity of the center of the bottom.

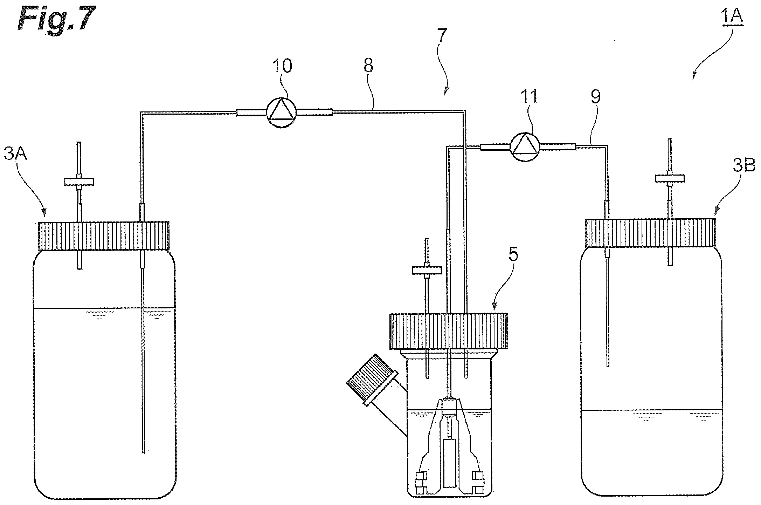

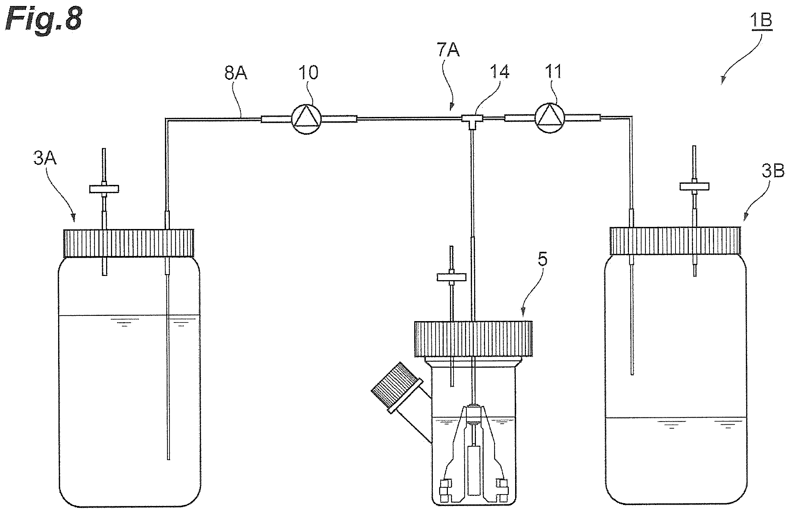

FIG. 7 and FIG. 8 are diagrams showing other embodiments of the cell culturing system including the cell culturing device. As shown in FIG. 7, a cell culturing system 1A includes the cell culturing device 5, a culture solution tank 3A, and a waste tank 3B. The culture solution tank 3A and the cell culturing device 5 are connected through the tube 8 provided with the feed pump 10. The cell culturing device 5 and the waste tank 3B are connected through the tube 9 provided with the feed pump 11. In the cell culturing system 1A, culture solution is supplied from the culture solution tank 3A to the cell culturing device 5, and the culture solution containing metabolites is supplied from the cell culturing device 5 to the waste tank 3B.

As shown in FIG. 8, a cell culturing system 1B includes the cell culturing device 5, the culture solution tank 3A, and the waste tank 3B. The culture solution tank 3A and the waste tank 3B are connected with the cell culturing device 5 through a feed circuit 7A. Specifically, a tube 8A is provided with a three-way stopcock 14. The culture solution is supplied from the culture solution tank 3A to the cell culturing device 5 through the three-way stopcock 14, and the culture solution containing metabolites is supplied from the cell culturing device 5 to the waste tank 3B through the three-way stopcock 14.

The three-way stopcock 14 switches channels depending on supply of the culture solution to the cell culturing device 5 and suction of the culture solution from the cell culturing device 5. That is, the three-way stopcock 14 establishes a channel between the culture solution tank 3 A and the cell culturing device 5 when culture solution is supplied from the culture solution tank 3A, and establishes a channel between the cell culturing device 5 and the waste tank 3B when culture solution is supplied to the waste tank 3B. In the cell culturing device 5, suction of the culture solution from the culture tank 15 and supply of the culture solution to the culture tank 15 are performed through the filter 21.

### Second Embodiment

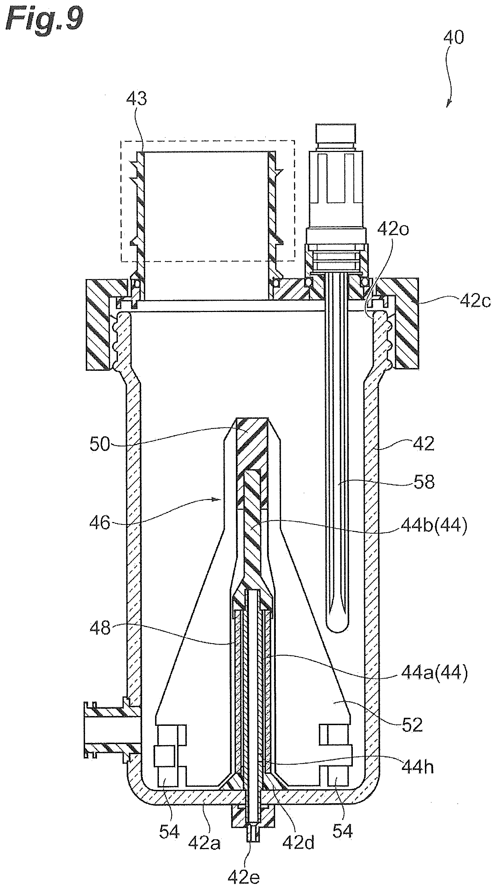

A second embodiment will now be described. FIG. 9 is a diagram showing a cross-sectional configuration of a cell culturing device according to the second embodiment. As shown in FIG. 9, a cell culturing device 40 includes a culture tank 42, a shaft member 44, a stirring mechanism 46, and a filter 48.

The culture tank 42 is preferably formed of a material that is inert to the culture solution components, does not have cytotoxicity, and is resistant to sterilization (including decontamination, disinfection, and asepsis) treatment. Examples of the material include glasses, synthetic resins, and stainless steels. The internal capacity, the shape, and the like of the culture tank 42 are appropriately determined according to the amount of the culture solution. In the present embodiment, the culture tank 42 is made of, for example, a synthetic resin and is shaped like a cylinder with a base. The culture tank 42 is provided with a lid 42c for closing an opening 42o provided on the top. The lid 42c is provided with, for example, an outlet 43 for drawing the culture solution containing cells or others, an air supply/exhaust port, and a pH sensor 58.

The shaft member 44 is supported by a bottom 42a of the culture tank 42. The shaft member 44 includes a conduit 44a and a support 44b. The conduit 44a is a linear member and has a hollow structure. The conduit 44a is preferably formed of a material that is inert to the culture solution components, does not have cytotoxicity, and is resistant to sterilization treatment. Examples of the material include synthetic resins and stainless steels. The conduit 44a is supported by the bottom 42a of the culture tank 42. Specifically, the conduit 44a is inserted through a support base 42d provided on the bottom 42a, and supported and fixed. The interior of the conduit 44a is in communication with a suction port 42e provided at the bottom 42a of the culture tank 42. At a side wall of the conduit 44a, an opening 44h is provided to communicate the interior with the exterior. Although FIG. 9 shows a single opening 44h, the number, the size, and the shape of the opening 44h can be appropriately set in accordance with the design.

The support 44b is provided on the top of the conduit 44a. The support 44b is disposed so as to close the opening at the upper end of the conduit 44a. The support 44b extends concentrically with the conduit 44a.

The stirring mechanism 46 includes an attachment 50 and stirring blades 52. The attachment 50 is rotatably attached to the support 44b of the shaft member 44. Specifically, the upper end of the support 44b is inserted in the attachment 50.

The stirring blades 52 are provided to be rotatable about the shaft member 44. Specifically, the attachment 50 is coupled to the upper ends of the stirring blades 52, and the attachment 50 is rotatable relative to the shaft member 44, whereby the stirring blades 52 rotate about the shaft member 44. The stirring blades 52 are preferably formed of a plate material inert and resistant to the culture solution. Examples of the material include thin plate-shaped synthetic resins and stainless steels (for example, SUS 316 having a thickness of 1 mm). The number of stirring blades 52 is set depending on the number of revolutions of the stirring mechanism 46. The stirring blades 52 are preferably disposed at regular intervals around the shaft member 44 in view of balance during stirring. Preferably, for example, two to four stirring blades 52 are provided. In the present embodiment, a configuration including two stirring blades 52 is illustrated.

Each of the stirring blades 52 is provided with a magnetic force element 54. The magnetic force element 54 is disposed at the lower end of the stirring blade 52. The magnetic force element 54 is, for example, a permanent magnet coated with, for example, tetrafluoroethylene. The magnetic force element 54 is fixed to a portion bent so as to surround the magnetic force element 54 at the lower end of the stirring blade 52. The stirring blade 52 is rotated by driving of the magnetic force element 54. Specifically, as shown in FIG. 5, the drive motor 34 is disposed below the culture tank 42 to drive the rotation of a pair of magnetic force elements 32 through the support 33. The stirring blades 52 rotate with the rotation of the magnetic force elements 32 disposed so as to face the magnetic force elements 54. The shaft of the drive motor 34 is disposed concentrically with the shaft member 44.

The filter 48 may be any filter that can separate the cells and others from the culture solution. The material, the shape, the number, and the like are not limited and can be appropriately set in accordance with the design. The filter 48 is, for example, porous, preferably, sintered and cylindrical. The filter 48 has the conduit 44a of the shaft member 44 inserted therethrough and is provided on the outer surface (outside) of the conduit 44a so as to cover the opening 44h of the conduit 44a. That is, the filter 48 is located inner than the movable region of the stirring blades 52 rotating about the shaft member 44 and is provided on the shaft member 44 independently of the stirring blades 52. In the cell culturing device 40, the culture solution is sucked from the culture tank 42 through the filter 48 and the interior of the conduit 44a.

As described above, the cell culturing device 40 in the present embodiment includes the filter 48 provided on the shaft member 44 configured to be unrotatable. The stirring blades 52 of the stirring mechanism 46 are provided to be rotatable about the shaft member 44. In this manner, in the cell culturing device 40, the stirring blades 52 rotate relative to the shaft member 44, whereas the shaft member 44 and the filter 48 do not rotate. Thus, in the cell culturing device 40, because the mechanism for sucking culture solution is not rotated, a simple configuration can be achieved. In the cell culturing device 40, the filter 48 sucking culture solution from the culture tank 42 is located inner than the movable region of the stirring blades 52 on the shaft member 44 about which the stirring blades 52 rotate. The culture solution is sucked from the culture tank 42 through the filter 48 and the interior of the shaft member 44. In the cell culturing device 40, disturbance by the filter 48 in the solution flow (laminar flow) produced by the rotation of the stirring blades 52 thus can be suppressed, thereby reducing stress on cells in connection with suction of the culture solution. In the cell culturing device 40, therefore, culturing of cells can be performed satisfactorily with a simple configuration.

In the present embodiment, the shaft member 44 is supported by the bottom 42a of the culture tank 42, and the shaft member 44 is inserted into the filter 48. In this way, in the cell culturing device 40, the mechanism for sucking culture solution is integrated to the shaft member 44, and the shaft member 44 does not rotate. Simplification of the device thus can be achieved.

The cell culturing device 40 according to the second embodiment may simply be configured such that the shaft member is supported by the bottom 42a of the culture tank 42. The configuration of the shaft member, the filter, and the stirring mechanism may be in different forms.

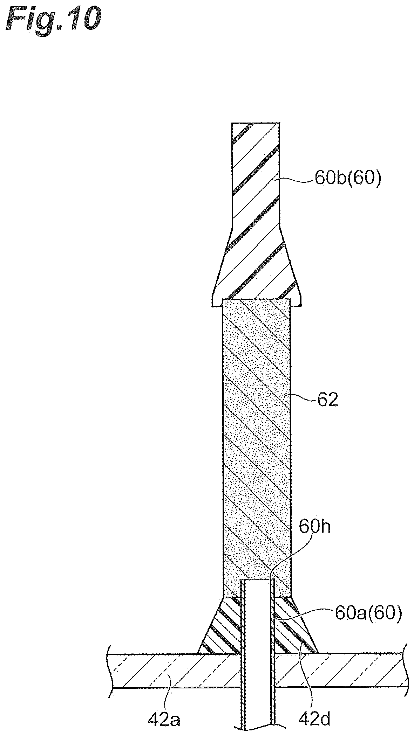

FIG. 10 to FIG. 14 are diagrams showing modifications of the cell culturing device according to the second embodiment. As shown in FIG. 10, a shaft member 60 includes a conduit 60a and a support 60b. The conduit 60a is a linear member and has a hollow structure. The conduit 60a is inserted through the support base 42d provided on the bottom 42a of the culture tank 42 and is supported by the bottom 42a of the culture tank 42. At the upper end of the conduit 60a, an opening 60h is provided. A filter 62 is provided on the conduit 60a so as to cover the opening 60h of the conduit 60a. Specifically, the filter 62 is shaped like a cylinder with a base and has the upper end of the conduit 60a inserted therein. That is, the conduit 60a has a configuration different from the conduit 44a in the foregoing embodiment and does not pass through the interior of the filter 62. The support 60b is provided on the upper end of the filter 62. The support 60b extends concentrically with the conduit 60a and the filter 62. The attachment 50 of the stirring mechanism 46 is attached to the support 60b.

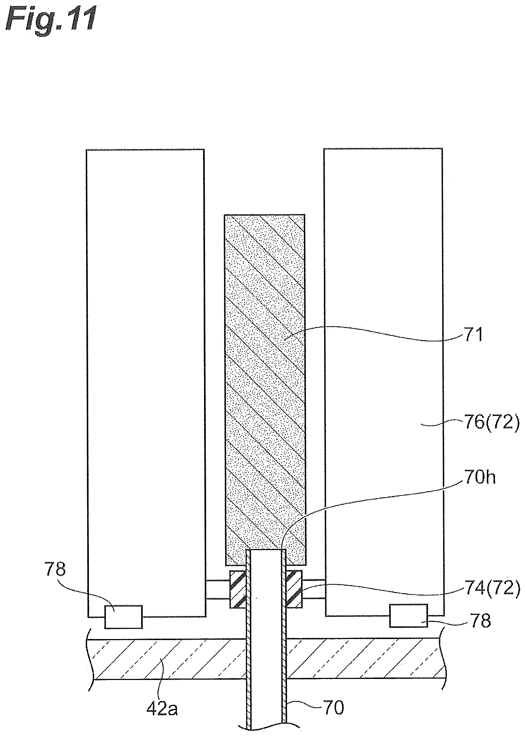

As shown in FIG. 11, a shaft member 70 is supported by the bottom 42a of the culture tank 42. At the upper end of the shaft member 70, an opening 70h is provided. A filter 71 is provided on the shaft member 70 so as to cover the opening 70h of the shaft member 70. Specifically, the filter 71 is shaped like a cylinder with a base and has the upper end of the shaft member 70 inserted therein. The stirring mechanism 72 includes an attachment 74 and stirring blades 76. The attachment 74 is rotatably attached to the shaft member 70. Specifically, the attachment 74 is attached to the shaft member 70 at a position below the lower end of the filter 71. The stirring blades 76 have their lower end sides coupled to the attachment 74 and are disposed with the filter 71 interposed therebetween.

Each of the stirring blades 76 is provided with a magnetic force element 78. The magnetic force element 78 is disposed at the lower end of the stirring blade 76. The magnetic force element 78 is, for example, a permanent magnet coated with, for example, tetrafluoroethylene. The stirring blade 76 is rotated by driving of the magnetic force element 78. Specifically, as shown in FIG. 5, the drive motor 34 is disposed below the culture tank 42 to drive the rotation of a pair of magnetic force elements 32 through the support 33. The stirring blades 76 rotate with the rotation of the magnetic force elements 32 disposed so as to face the magnetic force elements 78. The shaft of the drive motor 34 is disposed concentrically with the shaft member 70. The configuration of the stirring blades 76 is not limited to the configuration (shape) shown in FIG. 11.

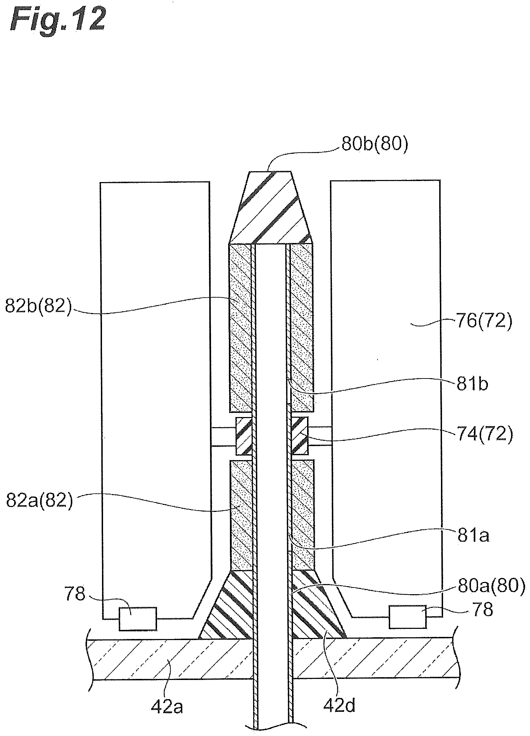

As shown in FIG. 12, a shaft member 80 includes a conduit 80a and a seal 80b. The conduit 80a is a linear member and has a hollow structure. The conduit 80a is inserted through the support base 42d provided on the bottom 42a of the culture tank 42 and is supported by the bottom 42a of the culture tank 42. At a side wall of the conduit 80a, openings 81a and 81b are provided to communicate the interior with the exterior. The openings 81a and 81b are separated from each other in the longitudinal direction of the shaft member 80 and are disposed corresponding to respective positions of a first filter 82a and a second filter 82b described later. Although FIG. 12 shows a single opening 81 a and a single opening 81b, the number, the size, and the shape of the openings 81a and 81b can be appropriately set in accordance with the design. The seal 80b seals (closes) the opening provided at the upper end of the conduit 80a.

The filter 82 includes a first filter 82a and a second filter 82b. That is, the filter 82 is divided into two parts. The first filter 82a and the second filter 82b each have a cylindrical shape. The first filter 82a has the lower end side of the conduit 80a inserted therethrough and is provided on the outer surface (outside) of the conduit 80a so as to cover the opening 81a. The second filter 82b has the upper end side of the conduit 80a inserted therethrough and is provided on the outer surface (outside) of the conduit 80a so as to cover the opening 81b. The upper end of the first filter 82a and the lower end of the second filter 82b are separated from each other with a predetermined spacing. The attachment 74 is disposed between the first filter 82a and the second filter 82b and is rotatably attached to the conduit 80a (shaft member 80). The stirring blades 76 are coupled to the attachment 74 and are disposed with the filter 82 interposed therebetween. The inner side of the lower end of the stirring blade 76 shown in FIG. 12 has a shape conforming to the shape of the support base 42d.

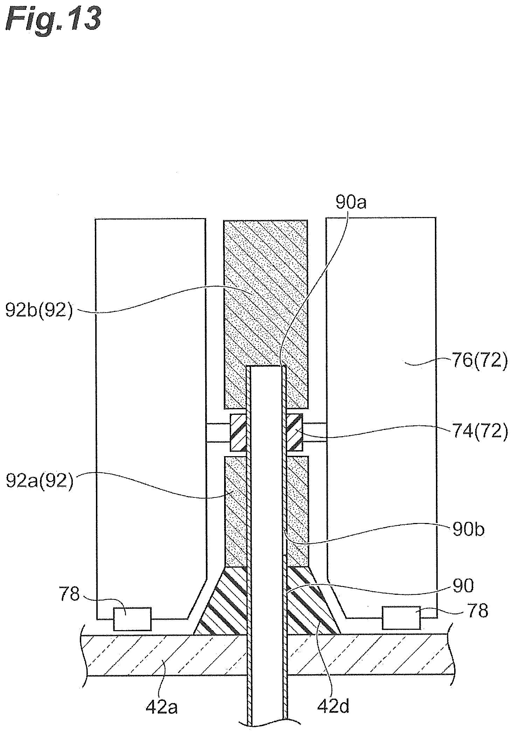

As shown in FIG. 13, a shaft member 90 is a linear member and has a hollow structure. The shaft member 90 is inserted through the support base 42d provided on the bottom 42a of the culture tank 42 and is supported by the bottom 42a of the culture tank 42. The shaft member 90 has an opening 90a at its upper end and has an opening 90b at the lower end side of the side wall to communicate the interior with the exterior. A filter 92 includes a first filter 92a and a second filter 92b. The first filter 92a has a cylindrical shape. The first filter 92a has the lower end side of the shaft member 90 inserted therethrough and is provided on the outer surface (outside) of the shaft member 90 so as to cover the opening 90b. The second filter 92b is provided on the shaft member 90 so as to cover the opening 90a of the shaft member 90. Specifically, the second filter 92b is shaped like a cylinder with a base and has the upper end of the shaft member 90 inserted therein. The upper end of the first filter 92a and the lower end of the second filter 92b are separated from each other with a predetermined spacing. Although FIG. 13 shows a single opening 90b, the number, the size, and the shape of the opening 90b can be appropriately set in accordance with the design. The attachment 74 is disposed between the first filter 92a and the second filter 92b and is rotatably attached to the shaft member 90. The stirring blades 76 are coupled to the attachment 74 and are disposed with the filter 92 interposed therebetween. The inner side of the lower end of the stirring blade 76 shown in FIG. 13 has a shape conforming to the shape of the support base 42d.

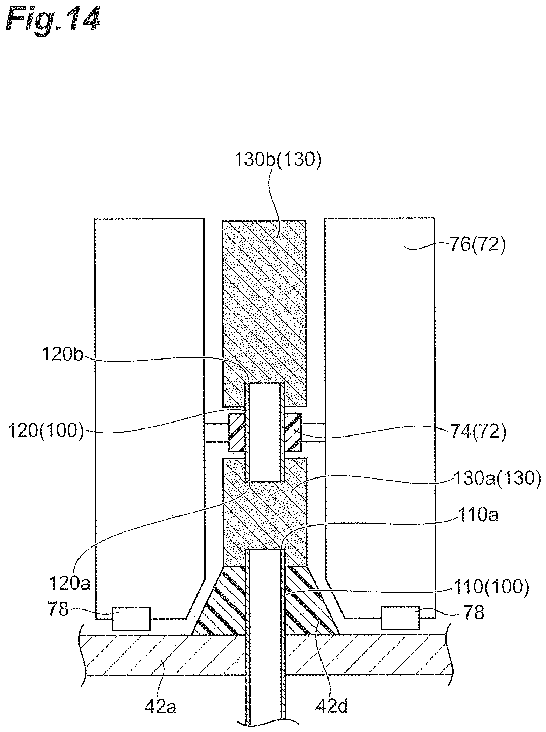

As shown in FIG. 14, a shaft member 100 has a first conduit 110 and a second conduit 120. The first conduit 110 and the second conduit 120 each are a linear member and have a hollow structure. The first conduit 110 is inserted through the support base 42d provided on the bottom 42a of the culture tank 42 and is supported by the bottom 42a of the culture tank 42. At the upper end of the first conduit 110, an opening 110a is provided. The second conduit 120 has an opening 120a and an opening 120b at its lower end and its upper end, respectively.

A filter 130 includes a first filter 130a and a second filter 130b. The first filter 130a is provided on the first conduit 110 and the second conduit 120 so as to cover the opening 110a of the first conduit 110 and the opening 120a of the second conduit 120. Specifically, the first filter 130a is shaped like a cylinder having insertion holes at both ends, and the upper end of the first conduit 110 and the lower end of the second conduit 120 are inserted in the insertion holes. The second filter 130b is provided on the second conduit 120 so as to cover the opening 120b of the second conduit 120. The second filter 130b is shaped like a cylinder with a base and has the upper end of the second conduit 120 inserted therein. The first filter 130a and the second filter 130b are coupled with each other through the second conduit 120 and are separated from each other with a predetermined spacing. The attachment 74 is disposed between the first filter 130a and the second filter 130b and is rotatably attached to the second conduit 120. The stirring blades 76 are coupled to the attachment 74 and are disposed with the filter 130 interposed therebetween. The inner side of the lower end of the stirring blade 76 shown in FIG. 14 has a shape conforming to the shape of the support base 42d.

The present invention is not limited to the foregoing embodiments and is susceptible of various modifications without departing from the spirit of the present invention.

In the foregoing embodiments, the stirring blades 27, 52, and 76 have been described by way of example. However, the shape of the stirring blades is not limited thereto. The shape of the stirring blades is preferably configured to achieve reduction in stress on cells.

### Examples

Although the present embodiments will be described in more detail with Examples below, the present embodiments are not limited to them.

### Example 1

### (Control Device for Cell Culturing System)

As a control device for the cell culturing system, an eight-stage animal culturing device BioJr.8 (BJR-25NA1S-8C, ABLE Corporation) was used. This control device alone can control eight cell culturing devices each having a capacity of 100 mL. The measurement and control items are stirring speed, temperature, pH, and dissolved oxygen concentration (DO). This control device can control each cell culturing device independently.

### (Preparation of Human iPS Cells)

Human iPS cells (253G1) were used as cultured cells. The cells were seeded in a culture dish (Corning Incorporated) coated with vitronectin (Life Technologies) and were cultured using Essential-8 as a culture solution. The culture solution was changed every day, and passaging was carried out once three to four days.

### (Culturing of Human iPS Cells)

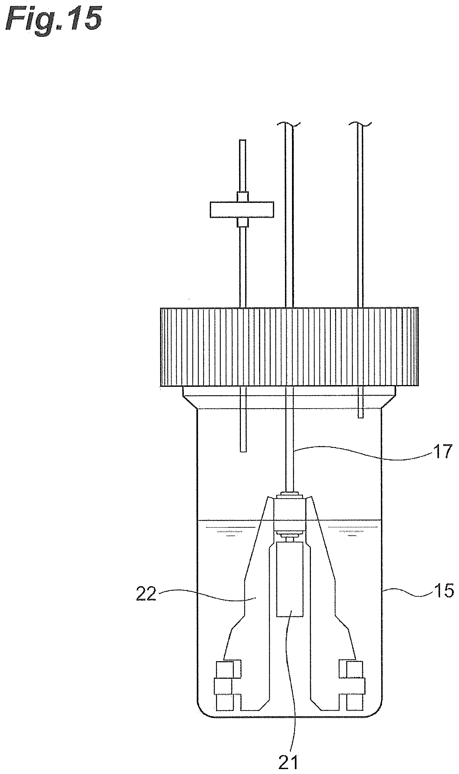

In Example 1, a cell culturing device shown in FIG. 15 was fabricated. A dedicated glass tank (ABLE Corporation) was used as the culture tank 15, and 100 mL of culture solution was used. The prepared human iPS cells were seeded with the number of cells 2×10⁷ (density of 2×10⁵/mL) to start culturing. This point of time was set as Day 0 of culture. As a culture solution, Essential-8 culture medium (Life Technologies) was used with addition of the following components. That is, 10 µM of Y-27632 (Wako Pure Chemical Industries, Ltd.) as ROCK (Rho-associated coiled-coil forming kinase/Rho-associated kinase) inhibitor, and 750 ng/mL of heparin (Sigma-Aldrich) were added. Stirring blades 22 were installed in the culture tank 15, and the number of revolutions was set to 60 rpm. A sensor (ABLE Corporation) capable of measuring temperature, pH, and dissolved oxygen concentration was installed in the culture tank 15. The dissolved oxygen concentration was set to be controlled to 40%. For this purpose, a gas introducing line was installed such that a gas mixture of oxygen, nitrogen, and air is ventilated through the upper surface of the culture solution in the culture tank 15. A discharge line for exhausting gas from the culture tank 15 was additionally installed. The temperature was set to 37°C.

A stainless steel hollow pipe (outer diameter of 3 mm and inner diameter of 1.8 mm) was used as the shaft member 17. A polyethylene sintered filter shaped like a cylinder with a base and having a diameter of 8 mm, a length of 15 mm, and a mean pore size of 30 µm was used as the filter 21. The shaft member 17 was fixed at the center of the lid of the culture tank 15 such that the culture solution passing through the filter 21 can be drawn from the culture tank 15 through the hollow portion of the shaft member 17. A port was installed, through which culture solution can be returned to the culture tank 15. The stirring blades 22 were installed so as to be rotatable around the shaft member 17. The filter 21 was installed at a height where the cylinder upper surface thereof is close to the level of the culture solution.

A sterile glass bottle having a capacity of 1 L was used as the component-controlling solution tank. A ventilation line, and inlet and outlet lines for culture solution were installed at the lid of the component-controlling solution tank. In the component-controlling solution tank, a culture solution component-controlling membrane was installed, which was formed by bundling 400 hollow fibers of Asahi polysulfone dialyzer APS (Asahi Kasei Medical Co., Ltd.) into an effective length of 20 cm and fixing them with urethane adhesive such that the lumen structure at both ends of the hollow fibers were open. The hollow fiber bundle was arranged in the interior of the component-controlling solution tank, and both ends of the hollow fiber bundle were connected with the inlet and outlet lines of culture solution through a circuit. As a component-controlling solution, 1 L of a solution of Y-27632 and heparin added to Essential-6 (Life Technologies) at the same concentrations as in the culture solution was used.

A silicone tube (inner diameter of 1 mm, outer diameter of 4 mm) was used as a feed circuit.

Two Perista pumps RP-23 (ABLE Corporation) were used as feed pumps for culture solution. The two pumps were connected to the feed circuit connecting the culture tank 15 with the component-controlling solution tank and were installed so as to be able to feed solution.

In the closed circuit, independently of the culture tank 15 and the component-controlling solution tank, 2 mL of a solution of 10 µg/mL of bFGF (Life Technologies) and 250 µg/mL of heparin (Sigma-Aldrich) dissolved in a culture solution DMEM/F12 (Life Technologies) was installed in a 10-mL syringe (TERUMO CORPORATION).

The cell culturing system shown in FIG 1 was fabricated by the method described above. A cell mass of iPS cells was produced by the method described below using the present system.

The circulation of the culture solution by the feed pumps was started on Day 1 of culture. The circulation speed was 100 mL/day on Day 1, 200 mL/day on Day 2, 400 mL/day on Day 3, and 600 mL/day on and subsequent to Day 4. The flow rates of the two pumps were finely adjusted such that the solution volume in the culture tank 15 was maintained substantially at the same level, and culturing was carried out while the culture solution was continuously perfused until Day 6. On Day 2 and Day 4 of culture, 1 mL of the mixed solution of bFGF and heparin was administered with the 10-mL syringe. On Day 6, the cell culturing was terminated. The cells were counted and the ratio of undifferentiation was measured as follows.

### (Counting of Cells)

On day 6, the cell mass was retrieved from the culture tank 15 and treated with a cell dissociation/suspension solution Accumax (Innovative Cell Technologies, Inc.) for 10 minutes into a single cell state. Dead cells were thereafter dyed by trypan blue, and living cells alone were counted using a hemocytometer.

### (Measuring of Undifferentiation Ratio)

In order to confirm that human iPS cells were kept undifferentiated, the positive rate of SSEA-4, which is an undifferentiation marker, was measured by flow cytometry. Anti-human/mouse SSEA-4 monoclonal antibody FAB1435F (R&D Systems, Inc.) was used as an antibody, and Cell Lab QuantaSC (Beckman Coulter, Inc.) was used as a measuring device. The positive rate of SSEA-4 was identified as the undifferentiation ratio.

### Example 2

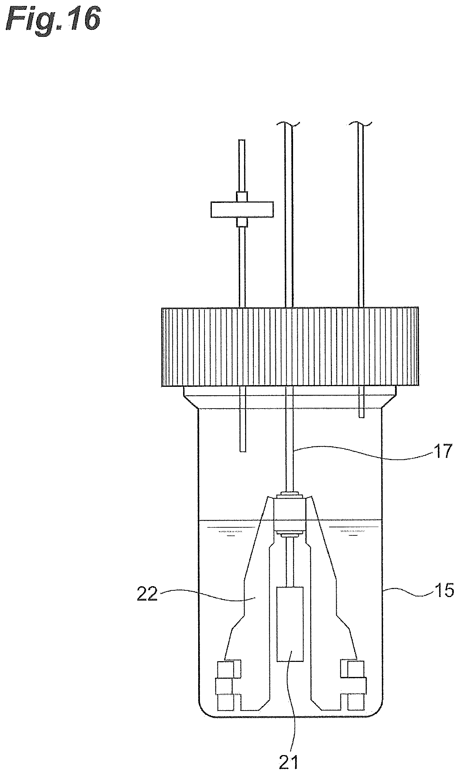

In Example 2, as shown in FIG. 16, the same structure as that of the cell culturing device in Example 1 was employed except that the installation height of the filter 21 was set at the intermediate level between the bottom surface of the culture tank 15 and the solution level height.

### Comparative Example 1

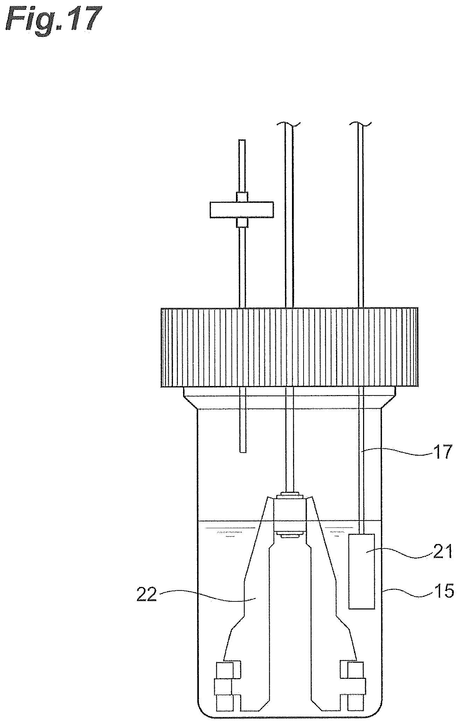

In Comparative Example 1, as shown in FIG. 17, the same structure as that of the cell culturing device in Example 1 was employed except that the filter 21 and the shaft member 17 were installed outside the rotation range of the stirring blades 22 and that the inlet line and the outlet line of culture solution were changed.

The culture results in Example 1, Example 2, and Comparative Example 1 above are shown in Table 1 below.

**[Table 1]**

| | Number of cells (×10⁸) | Undifferentiation ratio (%) |
|---|---|---|
| Example 1 | 5.0 | 98.7 |
| Example 2 | 5.8 | 99.1 |
| Comparative Example 1 | 0.2 | 99.4 |

As shown in Table 1, in the cell culturing device in Comparative Example 1, proliferation of the cells is not found, whereas the cell culturing devices in Example 1 and Example 2 achieve 20 or more fold proliferation compared with the start of culture. In both of the cell culturing devices in Example 1 and Example 2, the proportion of SSEA-4 positive undifferentiated cells is kept at an undifferentiation ratio as high as 95% or more. Based on the results, the method of the present embodiments is shown to be effective as the cell culturing device for human pluripotent stem cells.

### Reference Signs List

5, 40 ... cell culturing device, 15, 42 ... culture tank, 15c ... lid, 17, 44 ... shaft member, 17a, 17b, 17Aa ... opening, 17h, 44h ... opening, 19, 46 ... stirring mechanism, 21, 48 ... filter, 27, 52, 76 ... stirring blade, 42a ... bottom.

## Claims

1. A cell culturing device comprising:
a culture tank configured to accommodate culture solution containing a cell;
a shaft member at least partially disposed in the culture tank;
stirring means supported by the shaft member and disposed in the culture tank, the stirring means having a stirring blade configured to be rotatable about the shaft member; and
a filter disposed in contact with the shaft member, the filter allowing the culture solution to pass through and not allowing the cell to pass through, wherein
the shaft member is at least partially hollow, has an opening to introduce the culture solution to interior of the shaft member or to discharge the culture solution from the interior, and is configured to be unrotatable,
the filter is located inner than a movable region of the stirring blade rotating about the shaft member and is disposed on the shaft member independently of the stirring blade, and
suction of the culture solution from the culture tank and/or supply of the culture solution to the culture tank is performed through the filter and the interior of the shaft member.

2. The cell culturing device according to claim 1, wherein the shaft member is supported by a bottom of the culture tank.

3. The cell culturing device according to claim 2, wherein
the shaft member has a lower end having the opening and has an upper end closed,
the shaft member has a side surface having an opening in communication with the interior, and
the filter is disposed on an outer surface of the shaft member so as to cover the opening at the side surface.

4. The cell culturing device according to claim 3, wherein the filter has a tubular shape and extends on outside of the shaft member so as to cover the opening at the side surface.

5. The cell culturing device according to claim 2, wherein
the shaft member has a tubular shape that has a lower end having the opening and that has an upper end having an opening in communication with the interior, and
the filter is disposed on the shaft member so as to cover the opening at the upper end.

6. The cell culturing device according to claim 5, wherein the filter has a tubular shape with a base and extends on outside of the shaft member so as to cover the opening at the upper end.

7. The cell culturing device according to claim 1, wherein the shaft member is supported by a lid of the culture tank.

8. The cell culturing device according to claim 7, wherein
the shaft member has a tubular shape that has an upper end having the opening and that has a lower end having an opening in communication with the interior, and
the filter is disposed on the shaft member so as to cover the opening at the lower end.

9. The cell culturing device according to claim 8, wherein the filter has a tubular shape with a base and extends on outside of the shaft member so as to cover the opening at the lower end.

10. The cell culturing device according to claim 7, wherein
the shaft member has an upper end having the opening and has a lower end closed,
the shaft member has a side surface having an opening in communication with the interior, and
the filter is disposed on an outer surface of the shaft member so as to cover the opening at the side surface.

11. The cell culturing device according to claim 10, wherein the filter has a tubular shape and extends on outside of the shaft member so as to cover the opening at the side surface.

12. The cell culturing device according to any one of claims 1 to 11, wherein the filter is porous.
